# EUROPÄISCHE PATENTSCHRIFT

(11) **EP 2 063 916 B1**
(45) Veröffentlichungstag und Bekanntmachung des Hinweises auf die Patenterteilung: **19.11.2014**
(21) Anmeldenummer: 07800637.6
(22) Anmeldetag: 12.09.2007
(51) Int. Cl.: A61K 49/04

(54) **RÖNTGENKONTRASTMITTEL FÜR DIE POSTMORTALE, EXPERIMENTELLE UND DIAGNOSTISCHE ANGIOGRAPHIE**
X-RAY CONTRAST AGENT FOR POST-MORTEM EXPERIMENTAL AND DIAGNOSTIC ANGIOGRAPHY
AGENT DE CONTRASTE RADIOGRAPHIQUE POUR L'ANGIOGRAPHIE EXPÉRIMENTALE ET DE DIAGNOSTIC POSTMORTEM

(30) Priorität: 18.09.2006 CH 14852006; 08.02.2007 CH 2122007
(43) Veröffentlichungstag der Anmeldung: 03.06.2009
(60) Teilanmeldung: 12181686.2
(73) Patentinhaber: Forim-X AG, 5630 Muri (CH)
(72) Erfinder: GRABHERR, Silke, 1026 Denges (CH); GYGAX, Erich, 3011 Bern (CH)
(74) Vertreter: Pestalozzi, Deborah
(86) Internationale Anmeldenummer: PCT/CH2007/000446
(87) Internationale Veröffentlichungsnummer: WO 2008/034270

(56) Entgegenhaltungen:
- WO-A-92/18169
- WO-A-97/24064
- DE-B1- 2 602 907
- GB-A- 721 264
- GB-A- 2 014 043
- US-A- 2 675 343
- SAVAGE LABORATORIES: "Ethiodol (ethiodized oil) Injection" DAILY MED - INTERNET ARTICLE, [Online] XP002431180 Gefunden im Internet: URL:http://dailymed.nlm.nih.gov/dailymed/d rugInfo.cfm?id=1812> [gefunden am 2007-04-21]
- GRABHERR S. ET AL.: "Postmortem Angiography: Review of former and current Methods" FORENSIC RADIOLOGY REVIEW, Bd. 188, März 2007 (2007-03), Seiten 832-838, XP008078071
- GATES CORPORATION: "Viscosity Data" INTERNET ARTICLE, [Online] XP002431181 Gefunden im Internet: URL:http://www.gates.com/brochure.cfm?broc hure=2625&location_id=3046> [gefunden am 2007-04-21]
- J. BERMEYER: "Postmortale Koronangiographie und Perfusion normaler und pathologisch veränderter Herzen, Messung der Durchflusskapazität interkoronarer Anastomosen" BEITR. PATH. ANAT., Bd. 137, 1968, Seiten 373-390, XP008078238
- GRABHERRS. ET AL.: "Postmortem Angiography After Vascular Perfusion with Diesel Oil and lipophilic Contrast agent", AJR, vol. 187, 1 November 2006 (2006-11-01), pages W515-W523,
- S. Grabherr: "Postmortaler Kreislauf mit angiographischer Darstellung der arteriellen, kapillären und venoösen Strombahn", 1 April 2004 (2004-04-01), Universitätä Innsbruck, Innsbruck
- 'Fluids-Kinematic viscosities', [Online] Gefunden im Internet: <URL:http://www.engineeringtoolbox.com/kine matic-viscosity-d_397.html> [gefunden am 2013-04-19]
- 'Viscosity converting chart', [Online] Gefunden im Internet: <URL:http://www.engineeringtoolbox.com/visc osity-converter-d_413.html> [gefunden am 2013-04-03]
- 'Lipiodol ultra fluid product information', [Online] Gefunden im Internet: <URL:http://www.aspenpharma.com.au/product_ info/pi/Lipiodol_Ultra_Fluid_PI_14September 2012.pdf> [gefunden am 2013-04-19]
- "Micropaque Suspension; Dotarem", FACHINFORMATION DES ARZNEIMITTEL-KOMPENDIUM DER SCHWEIZ, 26 August 2011 (2011-08-26),
- 'Operationssaal', [Online] Gefunden im Internet: <URL:http://flexikon.doccheck.coni/de/Opera tionssaal> [gefunden am 2014-03-05]
- VDI-WÄRMEATLAS, no. 10, 1 January 2006 (2006-01-01), page Dca6,
- VDI-WÄRMEATLAS, no. 10, 1 January 2006 (2006-01-01), page Dca28,

## Beschreibung

### TECHNISCHES GEBIET

Die vorliegende Erfindung betrifft ein Kontrastmittel für die postmortale Angiographie, insbesondere zur Untersuchung von tierischen oder menschlichen Leichen oder Bestandteilen davon, z.B. von Gliedmassen oder von Organen. Das Kontrastmittel enthält dabei eine im wesentlichen ölbasierte, apolare Kontrastkomponente, wie sie für Röntgenuntersuchungen verwendet wird, wobei die Kontrastkomponente eine Kontrastkomponentenviskosität im Bereich von 30-100 mPas aufweist. Weiterhin betrifft die Erfindung angiographische Verfahren, in welchen ein derartiges Kontrastmittel wenigstens während Zeitabschnitten eingesetzt wird.

### STAND DER TECHNIK

Angiographie ist die Darstellung von Blutgefässen mittels Röntgenstrahlen. Hierzu wird ein Kontrastmittel, d. h. ein Stoff, der für Röntgenstrahlen kaum durchlässig ist, in das Blutgefäss injiziert. Auf dem Röntgenbild zeichnet sich dann der mit Kontrastmittel gefüllte Gefässinnenraum ab. Das resultierende Bild nennt man Angiogramm. In der Medizinersprache wird das lange Wort Angiografie oft mit dem Wort Angio abgekürzt. Zur Angiographie kann unter anderem auch Computertomographie eingesetzt werden. Die Computertomographie, Abkürzung CT, ist die rechnergestützte Auswertung einer Vielzahl aus verschiedenen Richtungen aufgenommener Röntgenaufnahmen eines Objektes, um ein dreidimensionales Bild zu erzeugen (Voxeldaten). Es handelt sich dabei um ein bildgebendes Verfahren.

Die derzeit in Verwendung stehenden Techniken zur bildgebenden Gefässuntersuchung lassen sich in Gruppen von Methoden einteilen: Einerseits stehen Ausgusstechniken zur Verfügung, andererseits gibt es Kontrastmittel, die in der Lage sind Kapillaren zu penetrieren. Eine allgemeine Übersicht über sämtliche Techniken der postmortalen Angiographie kann in "Postmortem Angiography - A Review of Former and Current Methods" (S. Grabherr et al, AJR 2006 in press) gefunden werden.

### Ausgusstechniken:

Bei den Ausgusstechniken werden beispielsweise Methylmethacrylate (zum Beispiel Mercox®), oder Microfil® eingesetzt. Diese Substanzen werden nach vorheriger Spülung der Gefässe frisch angemischt injiziert. Nach ihrer Aushärtung wird das Gewebe wegmazeriert, was 2 - 4 Wochen dauert. Übrig bleibt dann der Ausguss der Gefässe, der von blossem Auge, mit Hilfe eines Mikroskops oder Elektronemikroskops oder auch mittels Röntgentechniken untersucht werden kann (siehe Djonov et al, Anal Embryo/ 2000;202:347-357). Nachteile dieser Methode sind die aufwendige Herstellung dieser Präparate, die lange Zeitdauer, die das Mazerieren benötigt und nicht zuletzt die hohe Gefahr von Beschädigungen der Präparate durch das Mazerieren und das anschliessende Hantieren, bei dem sehr leicht kleine Stückchen des Präparates abbrechen können.

### Flüssige Kontrastmittel:

Die meisten flüssigen Konstrastmittel eignen sich nicht für Mikroangiographien. Auf Wasser basierende Mischungen haben die Eigenschaft, sehr schnell die Gefässe durch Penetration durch die Gefässwand zu verlassen (sogenannte Extravasation), was zu verwaschenen Konturen der dargestellten Gefässe führt. Auch ist ihr Kontrast nicht hoch genug, um kleine Gefässe wie Kapillaren klar darzustellen.

Ölige Kontrastmittel haben die Eigenschaft, innerhalb der Gefässe zu bleiben, ohne die Gefasswand zu penetrieren (normalerweise keine Extravasation). Dies führt zu einer deutlich konturierten Darstellung der Gefässe. Jedoch besteht keine Kapillargängigkeit aufgrund auftretender Verstopfungen der sogenannten Haargefässe.

Korpuskuläre Kontrastmittel können bei Verwendung kleiner korpuskulärer Partikel zur Microangiographie verwendet werden. Das bekannteste Kontrastmittel ist Micropaque ^{®}. Bei seiner Verwendung in Kombination mit Micro-CT-Geräten wurde jedoch das Problem einer Ausfällung der gelösten Partikel beschrieben, was zu Artefakten führt (M Marxen et al, Med Phys 2004,31:305-313). Marxen beschreibt in seinem Paper klar die Notwendigkeit und das Fehlen eines geeigneten Kontrastmittels für Microangiographie mittels Micro-CT. S. Grabherr beschreibt in der Dissertation "Postmortaler Kreislauf mit angiographischer Darstellung der arteriellen, kapillären und venösen Strombahn" aus dem Jahre 2004, die Verwendung von Diesel in einer Mischung mit Lipiodol ultrafluide zur postmortalen Bildgebung von Kälbergefässen.

### DARSTELLUNG DER ERFINDUNG

Der Erfindung liegt demnach die Aufgabe zugrunde, ein verbessertes Kontrastmittel für bildgebende Verfahren der eingangs genannten Art zur Verfügung zu stellen. Insbesondere geht es dabei also um die Verbesserung eines Kontrastmittels für die Angiographie, beispielsweise zur Untersuchung von tierischen oder menschlichen Leichen oder Bestandteilen davon , z.B. von Gliedmassen (welche auch noch am Körper sein können) enthaltend eine im wesentlichen apolare (mit anderen Worten so gut wie nicht oder gar nicht mit Wasser mischbare), das heisst in der Regel ölbasierte, Kontrastkomponente für Röntgenuntersuchungen. Die Kontrastkomponente verfügt dabei bei diesem Typ über eine Viskosität im Bereich von 30-100 mPas.

Problematisch an solchen apolaren öligen Kontrastkomponenten ist die Tatsache, dass sie eine hohe Viskosität aufweisen. Diese hohe Viskosität führt dazu, dass die Kontrastkomponente nicht beliebig in Kapillaren eindringen kann und entsprechend auch nicht das Blutgefässsystem vollständig abzubilden in der Lage ist. Zudem kann es sogar zu einer richtiggehenden Verstopfung von Kapillaren und Abschnitten führen. Der Vorteil der im wesentlichen nicht vorhandenen Extravasation wird dadurch bei vielen Anwendungen wieder rückgängig gemacht.

Überraschenderweise wurde festgestellt, dass es bei derartigen Kontrastkomponenten aber möglich ist, diese mit grossem Vorteil in einer Mischung zu verwenden. Die Mischung ist dabei dadurch gekennzeichnet, dass die Kontrastkomponente in einer Mischung mit wenigstens einer weiteren apolaren Komponente, deren Viskosität geringer ist oder höchstens gleich gross wie die Kontrastkomponentenviskosität, vorliegt. Die Zumischung dieser weiteren apolaren Komponente wird einerseits dazu verwendet werden, die Viskosität auf den gewünschten Wert, beispielsweise auf den Wert von Blut, einzustellen. Andererseits wird sie dazu verwendet, als Träger für die Kontrastkomponente zu wirken, ohne deren Eigenschaften hinsichtlich der Extravasation zu verändern. Unerwartet war dies insbesondere hinsichtlich der Tatsache, dass die positiven Eigenschaften bezüglich Extravasation beibehalten werden können und trotzdem die Viskosität der gesamten Mischung auf einen optimalen Wert eingestellt werden kann, und trotz Verdünnung der Kontrastkomponente ein hervorragender Kontrast möglich ist.

Bevorzugtermassen ist das Kontrastmittel im wesentlichen vollständig frei von Wasser, das heisst es liegt nicht als Emulsion vor. Es handelt sich mit anderen Worten bevorzugtermassen bei der erfindungsgemässen Mischung um eine Mischung, welche nur aus apolaren Komponenten besteht, wobei diese Komponenten bevorzugtermassen untereinander im wesentlichen beliebig mischbar sind.

Das Kontrastmittel ist ferner dadurch gekennzeichnet, dass die weitere apolare Komponente aus einem Alkan (bevorzugtermassen n-Alkan, iso-Alkan) mit einer Anzahl von Kohlenstoffatomen im Bereich von 12 - 16 besteht.

Bevorzugt werden so insbesondere Tetradekan öder Hexadekan. Bevorzugte Systeme besitzen einen Schmelzpunkt oberhalb von 0°C und/oder einen Siedepunkt von wenigstens 200°C.

Eine zusätzliche bevorzugte Ausführungsform zeichnet sich dadurch aus, dass die Kontrastkomponente eine Viskosität im Bereich von 30 - 80 mPas aufweist. So handelt es sich für die Angiographie beispielsweise bei der Kontrastkomponente bevorzugtermassen um ein iodbasiertes oder schwefelbasiertes Röntgenkontrastmittel, insbesondere bevorzugt um eine iodierte oder bromierte Komponente oder insbesondere Öl, beispielsweise um ein Propyliodon, einen Fettsäureethylester von iodiertem Mohnöl (z.B. Lipiodol), oder Iodipin, wobei es sich insbesondere bevorzugt um Lipiodol ultrafluid handelt.

Eine ganz besonders bevorzugte Ausführungsform der Mischung kennzeichnet sich dadurch, dass die weitere apolare Komponente eine Viskosität aufweist, welche niedriger ist als die Kontrastkomponentenviskosität. So verfügt bevorzugtermassen die weitere apolare Komponente über eine Viskosität im Bereich von 0.2 - 80 mPas, bevorzugt im Bereich von 2 - 60 mPas.

Wie bereits erläutert, ist es möglich, eine apolare Komponente mit einer vergleichsweise niedrigen Viskosität beizumischen, so bevorzugt mit einer Viskosität im Bereich von 1 - 10 mPas, bevorzugt im Bereich von 2 - 4 mPas. Besonders bevorzugt wird also beispielsweise eine binäre Mischung aus Lipiodol ultrafluide, und Tetradecan oder Hexadecan.

Bevorzugtermassen ist das Kontrastmittel dabei dadurch gekennzeichnet, dass das Volumen-Verhältnis von Kontrastkomponente zu weiterer apolarer Komponente im Bereich von 1:1 bis 1:10, bevorzugt im Bereich von 1:2 - 1:8, insbesondere bevorzugt im Bereich von 1:4 - 1:6 liegt.

Insbesondere für Microangio werden dabei Mischungen aus Kontrastkomponente und apolarer Komponente mit einer vergleichsweise niedrigen Viskosität (z. B. Tetradecan oder Hexadecan) eingesetzt in einem Verhältnis von 1: 4 oder 1: 6, wobei die Menge apolarer Komponente über den gewünschten Röntgenkontrast und die Eindringtiefe in kleine Kapillaren eingestellt wird.

Des Weiteren betrifft die vorliegende Erfindung ein Verfahren zur dynamischen angiographischen Untersuchung von tierischen oder menschlichen Leichen oder Organen, Bestandteilen, z.B. von Gliedmassen, davon. Das Verfahren ist bevorzugtermassen dadurch gekennzeichnet, dass ein Kontrastmittel, z.B. wie es oben beschrieben wurde, in das Blutgefässsystem eingebracht wird und anschliessend oder synchron eine Aufnahme unter Zuhilfenahme von Röntgenstrahlen, insbesondere eine CT-Aufnahme, vorgenommen wird.

Zudem betrifft die vorliegende Erfindung ein gewissermassen dynamisches Verfahren zur angiographischen Untersuchung von tierischen oder menschlichen Leichen oder Organen, Bestandteilen, z.B. von Gliedmassen davon. Dieses Verfahren ist dadurch gekennzeichnet, dass zunächst eine weitere apolare Komponente, wie sie oben geschildert wurde in das Blutgefässsystem im wesentlichen kontinuierlich eingebracht und darin zirkuliert wird, und während eines Zeitabschnitts dieser apolaren Komponente eine Kontrastkomponente, wie sie oben charakterisiert wurde, beigemischt wird, wobei anschliessend, synchron oder abschnittsweise Aufnahmen unter Zuhilfenahme von Röntgenstrahlen, insbesondere eine CT-Aufnahme, vorgenommen werden (so genannte dynamische Angiographie, zum Beispiel zunächst arterielle Bildgebung, dann parenchymale Bildgebung und dann venöse Bildgebung, wobei als Träger paraffinum perliquidum, ggf. in Mischung mit einem Decan (unter dem Begriff Decan sei in der Folge Tetradecan und/oder Hexadecan zu verstehen), eingesetzt wird und ein Stoss von Kontrastkomponente eingebracht wird). Generell ist es übrigens auch möglich, die weitere Komponente(n) einzufärben, so z.B. ist eine Rotfärbung des paraffinum perliquidum mit Sudanrot möglich.

Das Kontrastmittel zur Verwendung in diesem Verfahren kann also gemäss einer ersten bevorzugten Ausführungsform ein apolares Gemisch sein, wie es im Detail oben erläutert wurde.

Zudem betrifft die vorliegende Erfindung die Verwendung eines Kontrastmittels, wie es oben geschildert wurde, zur angiographischen Untersuchung von tierischen oder menschlichen Leichen oder Organen, Bestandteilen, z.B. von Gliedmassen davon.

Gemäss einem weiteren Ausführungsbeispiel wird ein Verfahren vorgeschlagen, welches dadurch gekennzeichnet ist, dass ein Kontrastmittel, bevorzugtermassen in Form einer Mischung, wie die oben beschrieben wurde, in nicht pulsierender Weise in die Blutgefässe eingebracht wird. Sämtliche Messungen am lebenden Körper müssen in pulsierender Weise zirkuliert werden, was nun an einer Leiche nicht erforderlich ist und wesentliche zusätzliche analytische Möglichkeiten eröffnet. Bevorzugtermassen wird das Kontrastmittel also unter im wesentlichen konstantem oder zeitlich mit wesentlich weniger oder wesentlich mehr als der natürlich möglichen Herzfrequenz variierendem Druck in die Blutgefässe eingebracht. Bevorzugtermassen wird also das Zirkulationsmedium nicht volumenkontrolliert sondern druckkontrolliert eingebracht.

Ein weiterer grosser analytischer Zusatznutzen, welcher aus den natürlichen Analysen nicht verfügbar ist, ist die Möglichkeit, das Kontrastmittel im Blutgefässsystem wenigstens abschnittsweise im Prozess entgegen der natürlichen Flussrichtung des Blutes zu zirkulieren. Auch werden, gegebenenfalls in Kombination mit der genannten Drucksteuerung und der Variation weiterer Parameter, welche am lebenden Körper nicht derart variiert werden können, unglaubliche zusätzliche analytische Möglichkeiten eröffnet. Generell kann zudem die Differenz bezüglich Volumen, Druck, Temperatur und/oder Komponentenkonzentration aus in das Kreislaufsystem eingeführtem Kontrastmittel und aus aus dem Kreislaufsystem austretendem Kontrastmittel registriert und in der Analyse bevorzugtermassen in quantitativer Weise mit berücksichtigt werden. So kann beispielsweise der Blutverlust durch eine Öffnung im Kreislaufsystem quantitativ ermittelt werden.

Generell ist es zudem oder alternativ möglich, das Kontrastmittel bei der Einführung in das Kreislaufsystem in wenigstens einem der folgenden Parameter während und/oder vor der Messung, insbesondere bevorzugt in Abhängigkeit der aufgenommenen Bilddaten, zu variieren: Temperatur, Mischungsverhältnis, Druck. Vor der Einführung von Kontrastmittel kann das Kreislaufsystem insbesondere bevorzugt unter Verwendung einer apolaren Komponente oder einer (physiologischen) NaCl- oder Lactatlösung, gespült werden.

Des weiteren offenbart die vorliegende Erfindung eine Vorrichtung zur Durchführung eines Verfahrens, wie es oben beschrieben wurde. Diese Vorrichtung ist bevorzugtermassen dadurch gekennzeichnet, dass sowohl Mittel zur pumpengetriebenen Einbringung von Kontrastmittel in das Kreislaufsystem vorgesehen sind als auch Mittel zur Aufnahme und quantitativen Erfassung des nach Zirkulation aus dem Kreislaufsystem austretenden Kontrastmittels.

Alternativ oder zusätzlich kann die Vorrichtung dadurch gekennzeichnet sein, dass eine Einheit angeordnet ist, mit welcher vor der Einbringung des Kontrastmittels in das Kreislaufsystem dieses entweder in automatisierter Weise in einem gewünschten Verhältnis aus Kontrastmittel und weiterer apolarer (oder polarer) Komponente(n), gegebenenfalls zeitabhängig, zugemischt und für die Einführung bereitgestellt wird und/oder das Kontrastmittel in der Temperatur eingestellt wird.

Weitere bevorzugte Ausführungsformen der vorliegenden Erfindung sind in den abhängigen Ansprüchen beschrieben.

### KURZE ERLÄUTERUNG DER FIGUREN

Die Erfindung soll nachfolgend anhand von Ausführungsbeispielen im Zusammenhang mit den Zeichnungen näher erläutert werden. Es zeigen:
- Fig. 1: einen postmortalen Mikro-CT Scan einer Maus mit Darstellung der grossen Gefässe;
- Fig. 2: eine bessere Übersicht über die grossen Gefässe des ganzen Körpers nach Entfernung der Rippen und Vorderbeine an der Workstation (virtuelles Skalpell);
- Fig. 3: eine Sichtbarmachung der kleineren Gefäss-Äste durch Veränderung der Fensterung an der Workstation;
- Fig. 4: eine Darstellung des Herzens und der Lebergefässe nach virtuellem Bearbeiten der Daten eines Teilkörperscans einer Maus;
- Fig. 5: eine Darstellung der Hauptäste der Nierengefässe nach virtuellem Ausschneiden des Organes aus einem Teilkörperscan;
- Fig. 6: einen hochauflösenden Scan einer Lungenprobe einer Maus mit 3D-Darstellung kleinster Blutgefässe;
- Fig. 7: eine bessere Sicht auf die Hauptäste der Lungengefässe einer Aufnahme nach Fig. 6 mit demselben Datensatz durch Veränderung der Fensterung;
- Fig. 8: verschiedene Darstellungsmöglichkeiten einer Niere aus dem Datensatz eines hochauflösenden Einzelorgan-Scans.

### WEGE ZUR AUSFÜHRUNG DER ERFINDUNG

Das vorgeschlagene Kontrastmittel kann beispielsweise zur mikroskopischen Gefässforschung eingesetzt werden (sog. Microangio).

Die bevorzugte Mischung für dieses Verfahren ist wie folgt:
Microangio: Lipiodol + Decan (Hexadecan oder Tetradecan) zum Beispiel im Verhältnis 1: 4

Nachdem wir im Rahmen der Entwicklung einer minimal invasiven postmortalen Angiographie erfolgreich ölige Perfusate und Kontrastmittel an Tierkadavern und menschlichen Leichen eingesetzt haben, wurde auf dieser Basis eine unerwartet vorteilhafte neue Mischung für den Einsatz einer postmortalen Angiographie mittels Micro-CT erstellt. Im Gegensatz zu unseren bisher verwendeten öligen Flüssigkeit kommt es bei dieser neuen Mischung nicht zu Mikroembolien des Kapillarsystems (S. Grabherr at al, A.IR 2006 in press), sondern zur Penetration und damit zur Darstellung dieses Gefässbereiches.

### Bestandteile:

1. Lipiodol ultrafluide® (Guerbert, France)
2. Tetradecan (Tetradecane olefine free, Fluka, Schweiz) oder Hexadecan (Hexadecane, Fluka, Schweiz)

Die Zusammensetzung dieser Komponenten kann variiert werden. Das ölige Kontrastmittel Lipiodol ultrafluide® sorgt für einen hohen Kontrast (ca. 2000 HU), während Decan als Verdünnungsmittel dient, welches die Kapillargängigkeit ermöglicht. Je mehr Decan verwendet wird, umso niedriger wird die Viskosität, und desto kleinere Gefässe können dargestellt werden.

Als bereits erfolgreich eingesetzte Mischungen ist ein Verhältnis von Lipiodol:Decan von 1:4 und 1:6 empfehlenswert.

### Vorteile des neuen Kontrastmittels:

Praktische Handhabungen: einer der wohl wesentlichsten Vorteile dieser Kontrastmittelmischung ist die praktische Handhabung. Es genügt eine einfache Injektion in den darzustellenden Gefässbereich.

Haltbarkeit der Proben: Da das ölige Kontrastmittel intravasal bleibt, können die Proben nach der Injektion mehrere Tage aufbewahrt und erst dann untersucht werden.

Transpörtfähigkeit der Proben: Die lange Haltbarkeit der Proben erlaubt den Transport zwischen Labors (z.B. Injektion in Bern, Untersuchung und Analyse in den USA).

Wiederholte Untersuchungsmöglichkeiten: Mehrfache Scans und Analysen einer Probe sind möglich, was bei unklaren Befunden essentiell ist.

3D-Rekonstruktion mit "Zoom in" und virtueller Probenbearbeitung: Mit Hilfe der Untersuchung mittels Micro-CT ist neben der zweidimensionalen auch eine dreidimensionale Rekonstruktion der Daten möglich. Durch Zoom in und virtuelles Schneiden der Proben können beliebige Areale vergrössert, ausgeschnitten und dargestellt werden, ohne die Proben zu zerstören.

Quantifizierung: Spezielle Software, welche z.B. zur Knochendichtemessung bereits für Micro-CT-Anwendungen existiert, kann sehr leicht zur Quantifizierung der kontrastreichen Gefässe angepasst werden.

Selektive, kaliberabhängige Darstellbarkeit: Je nach Mischungsverhältnis können verschiedene Gefässabschnitte entsprechend ihrem Kaliber dargestellt werden. So können selektiv nur grosse Versorgungsgefässe oder auch Kapillargefässe sichtbar gemacht werden.

Möglichkeit der dynamischen Angiographie: Bei entsprechender Injektionstechnik wird ein präzises Diskriminieren von arterieller, venöser und kapillärer Phase, in Analogie zur klinischen Angiographie erlaubt.

Wiederholbarkeit der Injektion: Bei Durchführung einer dynamischen Angiographie ist aufgrund des fehlenden Verlustes des Kontrastmittels in das umliegende Gewebe eine Ausspülung des Kontrastmittels aus dem Gefässsystem möglich, was eine erneute Injektion ohne Verfälschung der Ergebnisse durch Reste der vorherigen Injektion ermöglicht. Dies ist wichtig, wenn unklare Befunde in einer Phase der dynamischen Angiographie auftreten.

Möglichkeit weiterer Untersuchungen: Die untersuchten Organe können eingebettet und zusätzlich morphologisch untersucht werden (Paraffineinbettung und elektronenmikroskopische Schnittuntersuchung). Dies ist wichtig, wenn die 3D-Struktur mit der Gewebe-Morphologie korreliert und verglichen werden soll.

### Experimenteller Teil:

Mikroangiographie (Microangio):
bei diesem Verfahren wurde bei einer toten Maus jeweils eine Mischung aus Lipiodol und Decan als Kontrastmittel (KM) in das Gefässsystem injiziert und anschliessend wurde ein Mikro-CT mit einem Gerät des Typs "Siemens Micro-CT-Scanner" durchgeführt.

Vorversuch zur Testung des KM im "Lebend-Scanner:
KM (Lipiodol : Decan = 1:4) in 0,8 mm Venflon (Verweilkanüle) scannen: KM ist deutlich sichtbar.

### 1. Maus:

Von unten KM (Lipiodol : Decan = 1:4) in die V. cava inf.; Scan nur Oberkörper.

### 2. Maus:

Von unten KM (Lipiodol : Decan = 1:6) in die V. cava inf.; Kein Scan der kompletten Maus; Organentnahme für isolierte Organsscans:
- Herz
- Leber
- Niere
- Kopf

### 3. Maus:

Von unten KM (Lipiodol : Decan = 1:6) in die V. cava inf.; Injektion KM (Lipiodol : Decan = 1:6) in rechte V. saphena; 3 Scans für Ganzkörperdarstellung.

### 4. Maus:

Injektion KM (Lipiodol : Decan = 1:6) in linken Ventrikel; Scan nur Oberkörper.

### 5. Maus:

Injektion KM (Lipiodol Decan = 1:6) Aorta (wenig) und V. cava inf.; 2 Scans (Kopf und Thorax).

### 6. Maus:

Injektion KM (Lipiodol : Decan = 1:6) von unten in die V. cava inf.; 2 Scans (Kopf und Thorax).

### 7. Maus:

Injektion KM (Lipiodol : Decan = 1:5) in V. porta; Organentnahme für Einzelscans:
- Leberlappen (oberer inkl. Gallenblase)
- Herz
- Re u. li Niere

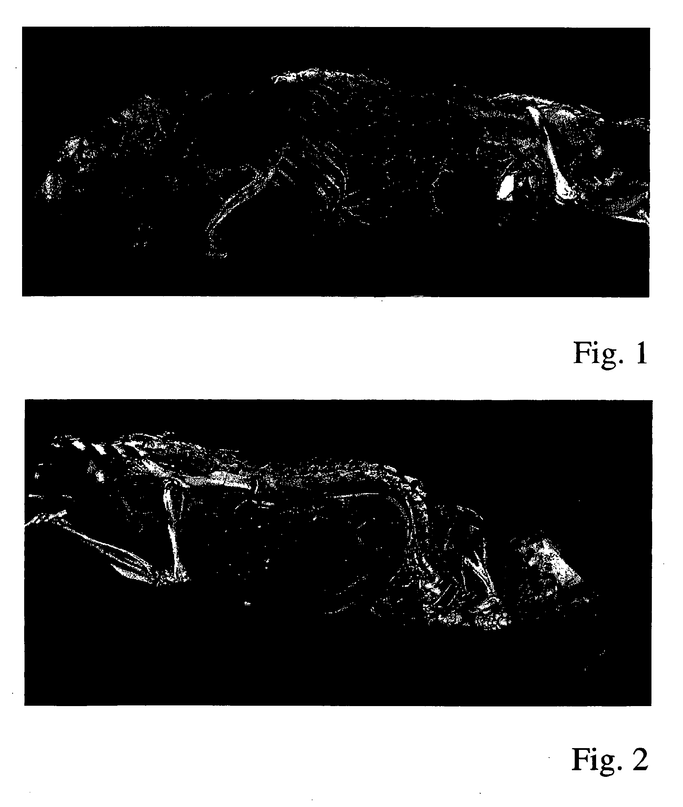

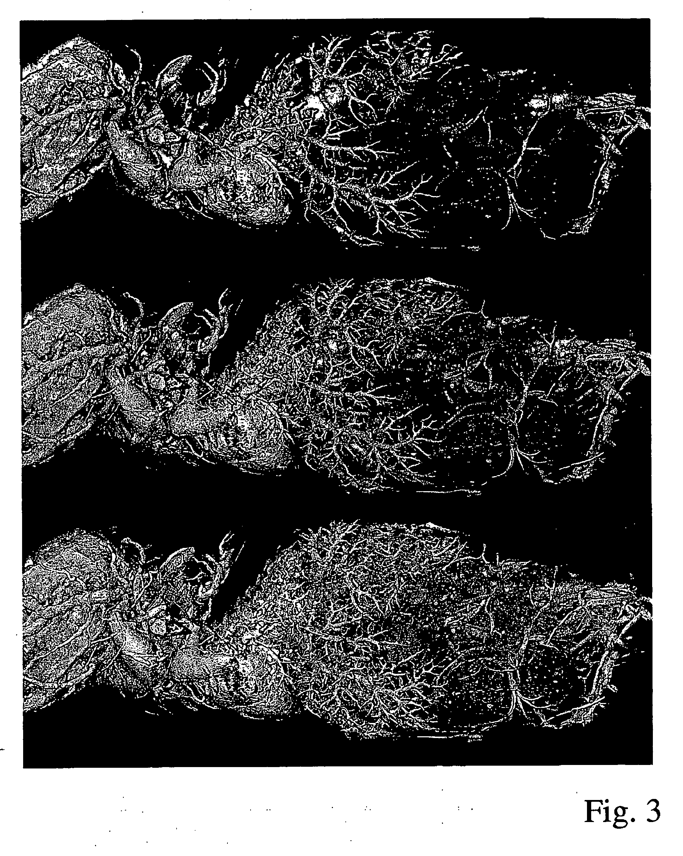

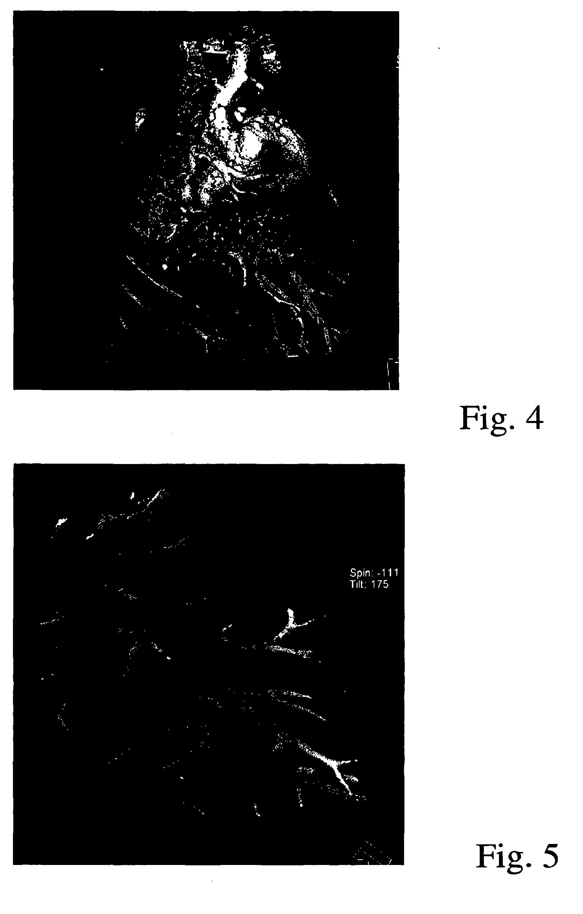

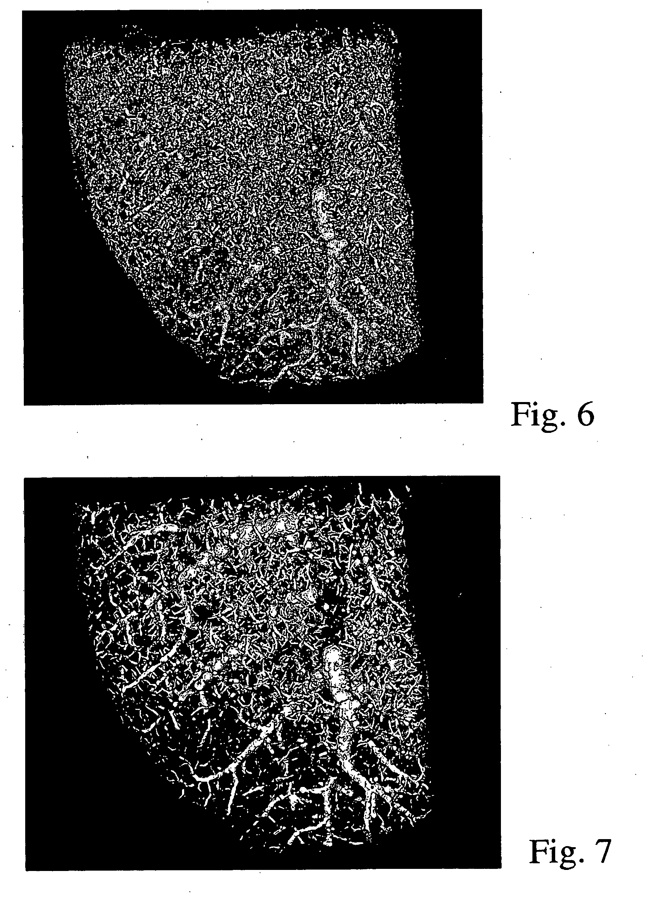

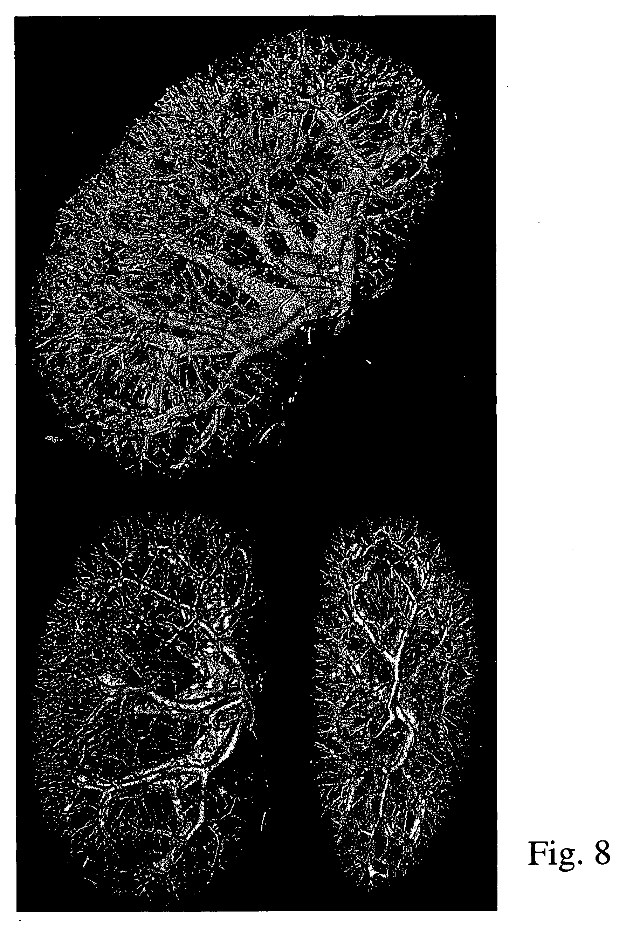

In den Figuren 1-8 sind unterschiedliche Rekonstruktionen aus dem Datensatz der oben angegebenen Micro-CT Scans dargestellt. Aus den Figuren ist ersichtlich, dass das Kontrastmittel einerseits eine hohe Auflösung erlaubt, und andererseits eine hervorragende Penetration selbst in kleinste Gefässsysteme erlaubt.

### Zusammenfassung:

Das neue Kontrastmittel erlaubt eine zuverlässige, rasche, wiederholte Untersuchung und Darstellung von 2D und 3D Gefässarchitektur in Labortieren (z.B. Microangiographie mit MicroCT). Zudem erlaubt sie dynamische Bildgebung und Quantifizierung des "Blutverlustes" mit der Angio-Perfusionsmethode. Praktische Handhabung, Quantifikationsmöglichkeiten, sowie exakte Darstellung verschiedenster Gefässabschnitte werden diese Methode als Standarduntersuchung bei der Evaluation genetisch-manipulierter Labortiere, pharmakologischen und toxikologischen Studien, sowie gentechnischen Produkten etablieren. Diese neue Visualisierungs- und Quantifikationsmethode soll die alten, zwar verlässigen, aber sehr zeitaufwendigen und hochspezifischen Techniken ablösen und den Weg für eine ausgedehnte, einfache, praktische und unkomplizierte Anwendung freimachen.

## Patentansprüche

1. Kontrastmittel für die Angiographie, insbesondere zur postmortalen, experimentellen und/oder diagnostischen Angiographie und zur Untersuchung von tierischen oder menschlichen Leichen oder Bestandteilen wie Gliedmassen oder Organen davon, enthaltend eine im wesentlichen ölbasierte, apolare Kontrastkomponente für Röntgenuntersuchungen, welche Kontrastkomponente eine Kontrastkomponentenviskosität im Bereich von 30-100 mPas aufweist,
**dadurch gekennzeichnet, dass**
die Kontrastkomponente in einer Mischung vorliegt mit nur einer weiteren apolaren Komponente, deren Viskosität geringer ist oder höchstens gleich gross wie die Kontrastkomponentenviskosität, wobei es sich bei der weiteren apolaren Komponente um ein Alkan mit 12-16 Kohlenstoffatomen handelt.

2. Kontrastmittel nach Anspruch 1, dass es sich bei der weiteren apolaren Komponente um ein n-Alkan handelt.

3. Kontrastmittel nach einem der vorhergehenden Ansprüche, **dadurch gekennzeichnet, dass** die Kontrastkomponente eine Viskosität im Bereich von 30 - 80 mPas aufweist, wobei es sich bei der Kontrastkomponente vorzugsweise um ein iodbasiertes oder schwefelbasiertes Röntgenkontrastmittel handelt, insbesondere bevorzugt um eine iodierte oder bromierte Komponente oder insbesondere Öl, beispielsweise um ein Propyliodon, einen Fettsäureethylester von iodiertem Mohnöl, oder Iodipin, wobei es sich insbesondere bevorzugt um Lipiodol ultrafluid handelt.

4. Kontrastmittel nach einem der vorhergehenden Ansprüche, **dadurch gekennzeichnet, dass** die weitere apolare Komponente eine Viskosität aufweist, welche niedriger ist als die Kontrastkomponentenviskosität, und vorzugsweise eine Viskosität im Bereich von 0.2 - 80 mPas aufweist, bevorzugt im Bereich von 2 - 60 mPas, insbesondere bevorzugt im Bereich von 1 - 10 mPas, am meisten bevorzugt im Bereich von 2 - 4 mPas.

5. Kontrastmittel nach einem der vorhergehenden Ansprüche, **dadurch gekennzeichnet, dass** das Volumen-Verhältnis von Kontrastkomponente zu weiterer apolarer Komponente im Bereich von 1:1 bis 1:10, bevorzugt im Bereich von 1:2 - 1:5, insbesondere bevorzugt im Bereich von 1:4 - 1:6 liegt.

6. Verfahren zur angiographischen Untersuchung von tierischen oder menschlichen Leichen oder Organen, Bestandteilen wie Gliedmassen davon, **dadurch gekennzeichnet, dass** ein Kontrastmittel nach einem der vorhergehenden Ansprüche, in das Blutgefässsystem eingebracht wird und anschliessend oder synchron Aufnahmen unter Zuhilfenahme von Röntgenstrahlen, insbesondere eine CT-Aufnahme, vorgenommen werden, wobei vorzugsweise das Kontrastmittel in nicht pulsierender Weise, bevorzugtermassen unter im wesentlichen konstantem oder zeitlich mit wesentlich weniger oder wesentlich mehr als der natürlich möglichen Herzfrequenz variierendem Druck in die Blutgefässe eingebracht wird, und/oder wobei vorzugsweise das Kontrastmittel im Blutgefässsystem wenigstens abschnittsweise im Prozess entgegen der natürlichen Flussrichtung des Blutes zirkuliert wird.

7. Verfahren nach Anspruch 6, **dadurch gekennzeichnet, dass** die Differenz bezüglich Volumen, Druck, Temperatur und/oder Komponentenkonzentration aus in das Kreislaufsystem eingeführtem Kontrastmittel und aus aus dem Kreislaufsystem austretendem Kontrastmittel registriert und in der Analyse bevorzugtermassen in quantitativer Weise mit berücksichtigt wird, und/oder dass vorzugsweise das Kontrastmittel bei der Einführung in das Kreislaufsystem in wenigstens einem der folgenden Parameter während und/oder vor der Messung, insbesondere bevorzugt in Abhängigkeit der aufgenommenen Bilddaten, variiert wird: Temperatur, Mischungsverhältnis, Druck, und/oder dass vorzugsweise vor der Einführung von Kontrastmittel das Kreislaufsystem insbesondere bevorzugt unter Verwendung einer apolaren Komponente oder einer NaCl- oder Lactatlösung, gespült wird.

8. Verfahren zur angiographischen Untersuchung von tierischen oder menschlichen Leichen oder Organen, Bestandteilen wie Gliedmassen davon, **dadurch gekennzeichnet, dass** zunächst eine weitere apolare Komponente nach einem der Ansprüche 1-5, in das Blutgefässsystem im wesentlichen kontinuierlich eingebracht und darin zirkuliert oder hindurchgeleitet wird, und während eines Zeitabschnitts dieser apolaren Komponente eine Kontrastkomponente nach einem der Ansprüche 1-5, insbesondere bevorzugt Lipiodol, oder eine polare Kontrastkomponente, beigemischt wird, wobei anschliessend, synchron oder abschnittsweise eine Aufnahme unter Zuhilfenahme von Röntgenstrahlen, insbesondere eine CT-Aufnahme, vorgenommen wird.

9. Verwendung eines Kontrastmittels nach einem der Ansprüche 1-5 zur angiographischen Untersuchung von tierischen oder menschlichen Leichen oder Organen, Bestandteilen wie Gliedmassen davon.

## Claims

1. Contrast agent for angiography, especially for postmortem, experimental and/or diagnostic angiography and for examination of animal or human corpses or components, such as extremities or organs thereof, comprising an essentially oil-based, non-polar contrast component for X-ray examinations, said contrast component having a contrast component viscosity in the range of 30-100 mPas, **characterized in that** the contrast component is present in a mixture with only one further non-polar component of a viscosity which is lower or at the most equal to the contrast component viscosity, wherein the further non-polar component is an alkane with 12-16 carbon atoms.

2. Contrast agent according to claim 1, **characterized in that** the further non-polar component is an n-alkane.

3. Contrast agent according to one of the preceding claims, **characterized in that** the contrast component has a vicosity in the range of 30-80 mPas, wherein the contrast component preferably is an iodine-based or sulphur-based radiographic contrast agent, especially preferably a iodized or brominated component or especially oil, for example a propyliodone, a fatty-acid-ethyl ester of iodized poppy-seed oil, or iodipine, wherein Lipiodol ultrafluid is especially preferred.

4. Contrast agent according to one of the preceding claims, **characterized in that** the further non-polar component has a viscosity which is lower than the viscosity of the contrast component and preferably has a viscosity of 0.2-80 mPas, preferably in the range of 2-60 mPas, more preferably in the range of 1-10 mPas, most preferably in the range of 2-4 mPas.

5. Contrast agent according to one of the preceding claims, **characterized in that** the volume-ratio of the contrast component to the further non-polar component is in the range of 1:1-1:10, preferably in the range of 1:2-1:5, especially preferably in the range of 1:4-1:6.

6. Method for the angiographic examination of animal or human corpses or organs, or components, such as extremities thereof, **characterized in that** a contrast agent according to one of the preceding claims is introduced into the vascular system and that subsequently or synchronously, radiographic images, especially a CT-image, are recorded by the aid of X-rays, wherein preferably the contrast agent is introduced into the blood vessels in a non-pulsing manner, preferably under an essentially constant pressure, or under a pressure which is temporally varying at a substantially lower or substantially higher rate than the naturally possible heart rate and/or wherein preferably the contrast agent is circulated in the vascular system at least section-wise during the process against the natural flow direction of the blood.

7. Method according to claim 6, **characterized in that** the difference of contrast agent introduced into the circulatory system and contrast agent exiting the circulatory system, with respect to volume, pressure, temperature and/or component concentration, is registered and taken into account, preferably quantitatively, in the analysis, and/or that preferably the contrast agent is varied during the introduction into the circulatory system in at least one of the following parameters during and/or prior to the measurement, especially preferably depending on the recorded image data: temperature, mixing ratio, pressure, and/or that preferably prior to the introduction of contrast agent, the circulatory system is rinsed, especially preferably by using a non-polar component or a NaCl- or lactate-solution.

8. Method for the angiographic examination of animal or human corpses or organs, or components, such as extremities thereof, **characterized in that** first a further non-polar component according to one of claims 1-5 is introduced into the vascular system in an essentially continuous manner, and is circulated therein or conducted through it, and that, during a time span of this non-polar component a contrast component according to one of claims 1-5, especially preferably Lipiodol, or a polar constrast component, is synchronously or section-wise admixed, wherein subsequently a radiograph image by the aid of X-rays, especially a CT-image, is recorded.

9. Use of a contrast agent according to one of claims 1-5, for the angiographic examination of animal or human corpses or organs, or components, such as extremities thereof.

## Revendications

1. Agent de contraste pour l'angiographie, en particulier pour l'angiographie post-mortem, expérimentale et/ou diagnostique et pour l'examen de cadavres animaux ou humains, ou de constituants, tels que des membres ou des organes de ceux-ci, contenant un composant de contraste apolaire, essentiellement à base d'huile pour des examens aux rayons X, le composant de contraste présentant une viscosité de composant de contraste dans la plage de 30-100 mPas, **caractérisé en ce que** le composant de contraste se trouve dans un mélange avec seulement un autre composant apolaire, dont la viscosité est inférieure ou au maximum égale à la viscosité du composant de contraste, l'autre composant apolaire étant un alcane comprenant 12-16 atomes de carbone.

2. Agent de contraste selon la revendication 1, en ce que qu'il s'agit, pour l'autre composant apolaire, d'un n-alcane.

3. Agent de contraste selon l'une quelconque des revendications précédentes, **caractérisé en ce que** le composant de contraste présente une viscosité dans la plage de 30-80 mPas, le composant de contraste étant de préférence un agent de contraste pour rayons X à base d'iode ou à base de soufre, en particulier de préférence un composant ou en particulier une huile iodé(e) ou bromé(e), par exemple d'une propyliodone, d'un ester éthylique d'acide gras d'huile d'oeillette iodée, ou d'Iopidine et en particulier de préférence de Lipiodol ultrafluide.

4. Agent de contraste selon l'une quelconque des revendications précédentes, **caractérisé en ce que** l'autre composant apolaire présente une viscosité qui est inférieure à la viscosité du composant de contraste et présente de préférence une viscosité dans la plage de 0,2-80 mPas, de préférence dans la plage de 2-60 mPas, en particulier de préférence dans la plage de 1-10 mPas, le plus préférablement dans la plage de 2-4 mPas.

5. Agent de contraste selon l'une quelconque des revendications précédentes, **caractérisé en ce que** le rapport volumique du composant de contraste à l'autre composant apolaire se situe dans la plage de 1:1 à 1:10, de préférence dans la plage de 1:2-1:5, en particulier de préférence dans la plage de 1:4-1:6.

6. Procédé pour l'examen angiographique de cadavres animaux ou humains, ou d'organes, de constituants tels que des membres de ceux-ci, **caractérisé en ce qu'**un agent de contraste selon l'une quelconque des revendications précédentes est introduit dans le système vasculaire et on réalise ensuite ou de manière synchrone des clichés à l'aide de rayons X, en particulier un cliché de TDM, l'agent de contraste étant de préférence introduit dans les vaisseaux dans un mode non pulsé, plus préférablement sous une pression essentiellement constante ou variant dans le temps de manière sensiblement moins ou sensiblement plus que la fréquence cardiaque naturellement possible et/ou l'agent de contraste étant de préférence mis en circulation dans le système vasculaire dans le procédé au moins par sections en sens inverse du sens d'écoulement normal du sang.

7. Procédé selon la revendication 6, **caractérisé en ce que** la différence en ce qui concerne le volume, la pression, la température et/ou la concentration en composants entre l'agent de contraste introduit dans le système circulatoire et l'agent de contraste sortant du système circulatoire est enregistrée et prise en considération dans l'analyse, de préférence de manière quantitative, et/ou **en ce qu'**au moins un des paramètres de l'agent de contraste est de préférence varié, lors de l'introduction dans le système circulatoire, pendant et/ou avant la mesure, en particulier de préférence en fonction des données d'images enregistrées : température, rapport de mélange, pression, et/ou **en ce que** le système circulatoire est de préférence rincé avant l'introduction de l'agent de contraste, en particulier de préférence en utilisant un composant apolaire ou une solution de NaCl ou de lactate.

8. Procédé pour l'examen angiographique de cadavres animaux ou humains, ou d'organes, de constituants tels que des membres de ceux-ci, **caractérisé en ce qu'**on introduit d'abord un autre composant apolaire selon l'une quelconque des revendications 1-5 dans le système vasculaire de manière essentiellement continue et on le met en circulation ou on le guide à travers celui-ci, et pendant un intervalle de temps on mélange à ce composant apolaire un composant de contraste selon l'une quelconque des revendications 1-5, en particulier de préférence du Lipiodol ou un composant de contraste polaire, un cliché étant réalisé consécutivement, de manière synchrone ou par sections à l'aide de rayons X, en particulier un cliché de TDM.

9. Utilisation d'un agent de contraste selon l'une quelconque des revendications 1-5 pour l'examen angiographique de cadavres animaux ou humains, ou d'organes, de constituants tels que des membres de ceux-ci.
